# EUROPEAN PATENT SPECIFICATION

(11) **EP 2 231 627 B1**
(45) Date of publication and mention of the grant of the patent: **01.06.2011**
(21) Application number: 08857548.5
(22) Date of filing: 02.12.2008
(51) Int. Cl.: C07D 277/04, C07D 317/22

(54) **NEW PROCESS FOR THE SYNTHESIS OF MOGUISTEINE**
NEUES VERFAHREN ZUR SYNTHESE VON MOGUISTEIN
NOUVEAU PROCÉDÉ POUR LA SYNTHÈSE DE MOGUISTÉINE

(30) Priority: 03.12.2007 EP 07425770
(43) Date of publication of application: 29.09.2010
(73) Proprietor: A.M.S.A. ANONIMA MATERIE SINTETICHE E AFFINI S.p.A., 20148 Milano (IT)
(72) Inventor: VIGANO', Enrico, I-22040 Lurago D'erba (IT); ARRIGHI, Massimiliano, I-22070 Luisago (IT); MOLTENI, Renato, I-22044 Inverigo (IT); LANFRANCONI, Simona, I-22070 Montano Lucino (IT); LANDONIO, Ernesto, I-20027 Rescaldina (IT)
(74) Representative: Cattaneo, Elisabetta
(86) International application number: PCT/EP2008/066591
(87) International publication number: WO 2009/071528

(56) References cited:
- EP-A- 0 169 581
- EP-A- 0 333 080
- GANDOLFI C A ET AL: "N-ACYL-2-SUBSTITUTED-1,3-THIAZOLIDINES, A NEW CLASS OF NON-NARCOTIC ANTITUSSIVE AGENTS: STUDIES LEADING TO THE DISCOVERY OF ETHYL 2- not (2-METHOXYPHENOXY)METHYL 3/4 -BETA-OXOTHIAZOLIDINE-3-PROPANOATE" JOURNAL OF MEDICINAL CHEMISTRY, AMERICAN CHEMICAL SOCIETY. WASHINGTON, vol. 38, no. 3, 1 January 1995 (1995-01-01), pages 508-525, XP002909416 ISSN: 0022-2623

## Description

### FIELD OF THE INVENTION

The present invention concerns a new process for the synthesis of ethyl ester of (R,S)-3-[2-[(2-methoxyphenoxy)methyl]-1,3-thiazolidin-3-yl]-3-oxypropanoic acid, of formula (1). 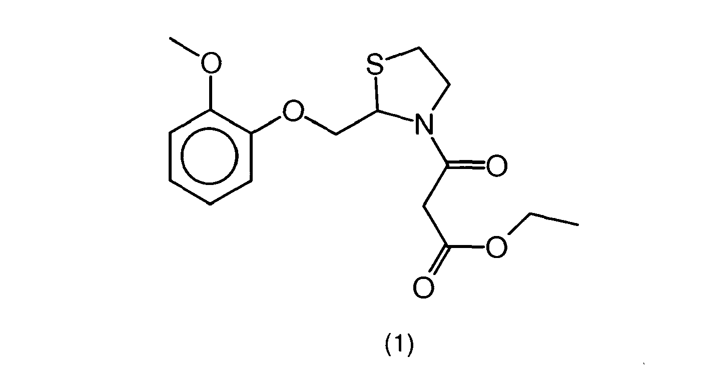 commonly known as moguisteine. The invention also relates to a new intermediate named as of 2-[(2-methoxyphenoxy)methyl]-1,3-dioxolane (4).

### STATE OF THE ART

Moguisteine is a peripheral antitussive agent which has shown powerful effects in the treatment of non-productive coughs, generally associated with respiratory disorders of varying severity.

Compounds with the 3-acyl-2-substituted-thiazolidine structure were described for the first time in EP 169581 B1 as agents having antitussive activity.

Moguisteine was prepared for the first time and described as the compound of choice within the 3-acyl-2-substituted-thiazolidine family in EP 333080 B1.

According to the teachings given in said patent, N-acyl-substituted thiazolidines are prepared where the acyl is a beta-carbonyl residue which is responsible for their activity being greater and more potent than the other substituted N-acyl-thiazolidines of EP 169581 B1.

In EP 333080 B1 moguisteine is prepared in example 1 via a process that comprises the reaction between (R,S)-2-(2-methoxy-phenoxymethyl)-1,3-thiazolidine and ethyl malonyl chloride. Specifically, an aqueous solution of KHCO₃ is firstly added to a solution of (R,S)-2-(2-methoxy-phenoxymethyl)-1,3-thiazolidine in ethyl acetate then, after cooling, an ethyl malonyl chloride solution is added drop-wise. The compound of interest is obtained by crystallization from the oily residue of the organic phase.

Moguisteine synthesis by way of the ethyl malonyl chloride reagent is repeated and further detailed by the same inventors of EP 333080 B1 in the article titled: "N-Acyl-2-substituted-1,3-thiazolidines, a new class of non-narcotic antitussive agents: studies leading to the discovery of ethyl 2-[(2-methoxyphenoxy)methyl]-β-oxothiazolidine-3-propanoate" (J.Med.Chem 1995, 38, 508-525), in which moguisteine is indicated as the most effective and safe antitussive agent in the family of N-Acyl-2-substituted-1,3-thiazolidine compounds.

In said article, moguisteine is obtained by a process that initially comprises the formation of (R,S)-2-(2-methoxy-phenoxymethyl)-1,3-thiazolidine then reacting thiazolidine obtained with ethyl malonyl chloride, in a similar manner to the example in EP 333080 B1. The process of (R,S)-2-[(2-methoxy-phenoxy)methyl]-1,3-thiazolidine formation indicated in the article is carried out in two steps:
1) formation of 2-(2-methoxyphenoxy)acetaldehyde diethyl acetal by reaction at 150°C of a guaiacol (2-methoxyphenol) compound and 2-bromoacetaldehyde diethyl acetal of formula: 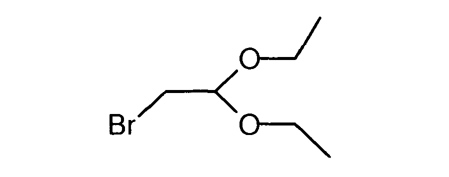 in N-methylpyrrolidone and in the presence of K₂CO₃ followed by distillation; and
2) synthesis of (R,S)-2-(2-methoxy-phenoxymethyl)-1,3-thiazolidine by reaction of 2-(2-methoxyphenoxy)acetaldehyde diethyl acetal with cysteamine hydrochloride in a mixture of ethanol/water/HCl, subsequent addition of a NaOH solution and recrystallization of the obtained precipitate from ethanol/water.

(R,S)-2-(2-methoxy-phenoxymethyl)-1,3-thiazolidine obtained in this manner is then reacted with ethyl malonyl chloride in a biphasic system of ethyl acetate/water in the presence of potassium bicarbonate to obtain a reaction product which is washed, dried, decolorized and concentrated under vacuum. The moguisteine is obtained from the concentrate diluted with hexane, after filtering and washing, as a recrystallization product from ethanol/water mixture.

The known processes for preparing moguisteine however comprise reacting substituted thiazolidine with ethyl malonyl chloride.

Ethyl malonyl chloride is known to be a poorly stable and rather costly compound. It is obtained by means of a rather long synthesis scheme which uses the formation of monoethylmalonic acid (obtained from the alkaline hydrolysis of diethyl malonic ester and subsequent acidification by displacement of salt), which must be reacted with thionyl chloride in the presence of dichloromethane to give ethyl malonyl chloride. Moreover, the product obtained requires a purification by distillation under vacuum, which must be carried out with extreme caution as ethyl malonyl chloride is unstable to heat and tends to decompose. The quality of the final product, i.e. ethyl malonyl chloride, is consequently never higher than 95%. The need was therefore felt for a process which avoids using the ethyl malonyl chloride reagent and leads to the formation of moguisteine at high purity. According to the known process, (R,S)-2-(2-methoxy-phenoxymethyl)-1,3-thiazolidine is obtained by reacting guaiacol with bromoacetaldehyde diacetal. Following the details of the prior art documents, the inventors of the present invention repeated said reaction and discovered that:
- an undesired hydrolysis can occur to give the free aldehyde and ethanol, which by reaction with guaiacol can give the by-product 2-ethoxyanisole;
- the intermediate 2-(2-methoxyphenoxy)acetaldehyde diethyl acetal obtained from the reaction is a black liquid containing polycondensation compounds which have the same reactivity as 2-(2-methoxyphenoxy)acetaldehyde diethyl acetal when released.

It is therefore evident that using the known intermediate as such in reaction step 2) leads to a thiazolidine and subsequently to moguisteine with a high impurity content. Furthermore, the high molecular weight impurities are hard to remove by the usual purification processes and require distillation at high vacuum (114°C, 0.5 mm Hg).

Object of the invention is therefore to provide a process that leads to the formation of moguisteine at high yields and high purity, while being inexpensive and easily achievable for industrial scale production.

### SUMMARY OF THE INVENTION

Such an object was achieved via a moguisteine synthesis process which comprises the following steps:
a) reacting guaiacol (2) with 2-Xmethyl-1,3-dioxolane (3) wherein X is a leaving group, to obtain 2-[(2-methoxyphenoxy)methyl]-1,3-dioxolane (4): 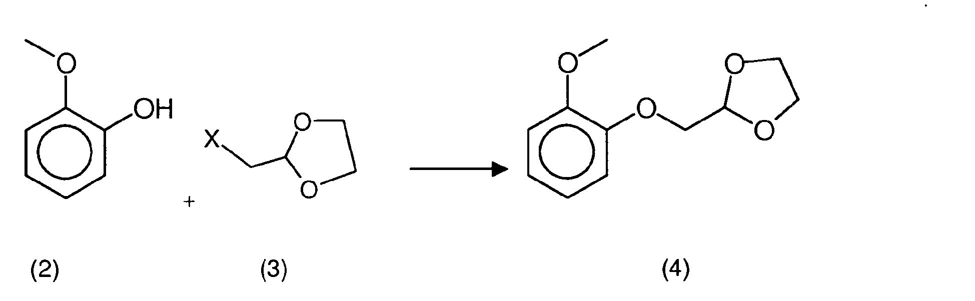
b) reacting the obtained 2-[(2-methoxyphenoxy)methyl]-1,3-dioxolane (4) with cysteamine (5) in the presence of an acid to obtain (R,S)-2-[(2-methoxyphenoxy)methyl]-1,3-thiazolidine (6): 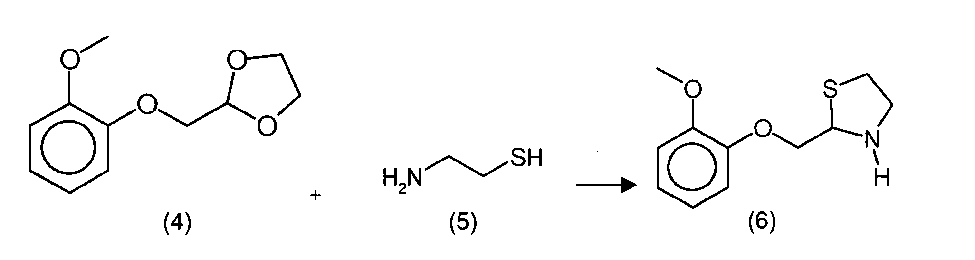
c) reacting (R,S)-2-[(2-methoxyphenoxy)methyl]-1,3-thiazolidine (6) with monoethylmalonic acid (7) or a salt thereof, in the presence of a condensing agent, to obtain moguisteine (1):

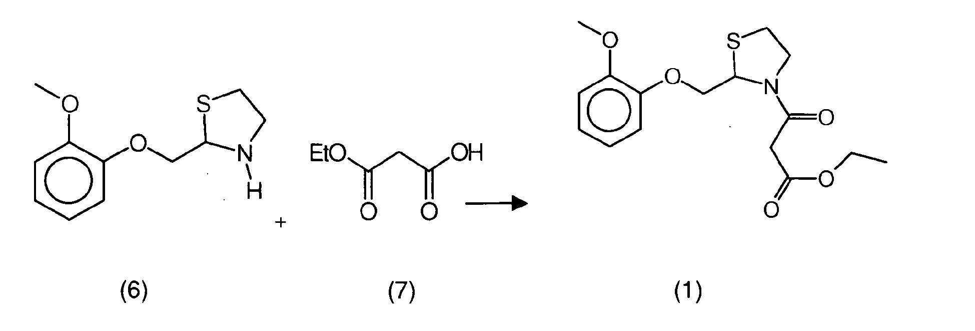

The process of the invention allows the compound 2-[(2-methoxyphenoxy)methyl]-1,3-dioxolane (4), being a solid with excellent stability and crystalline characteristics, to be easily separated and with good purity at the end of step a). In accordance with another aspect, the invention also concerns the intermediate 2-[(2-methoxyphenoxy)methyl]-1,3-dioxolane of formula (4):

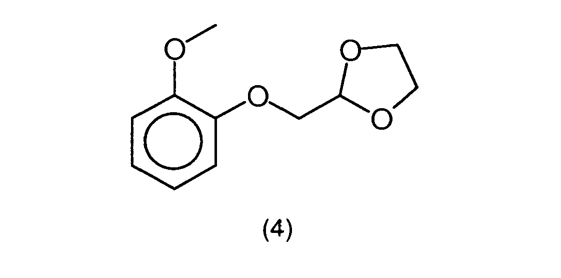

In the present invention the moguisteine molecule of formula (1) 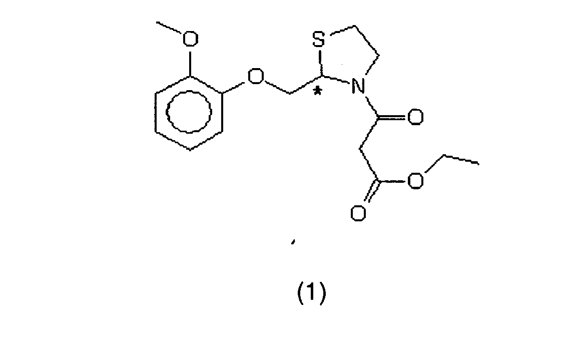 comprises in its definition any stereochemical configuration associated to the chiral centre in the formula itself, comprising the racemic mixture and enantiomers obtainable by separation techniques known to the expert of the art.

### DESCRIPTION OF THE FIGURES

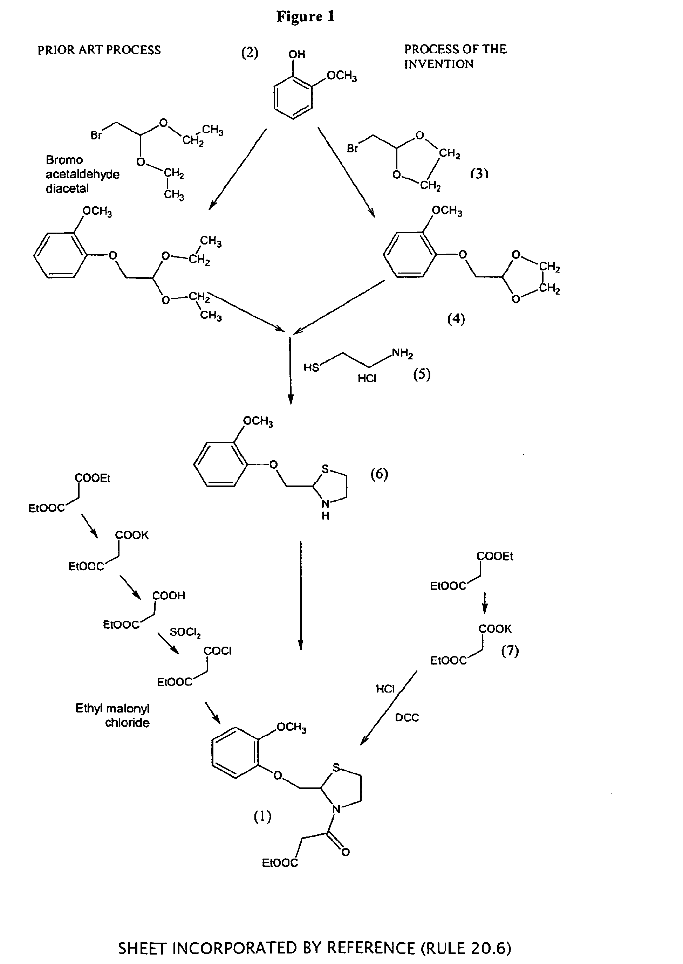

Figure 1 is a scheme comparing the known process for obtaining moguisteine comprising the use of bromoacetaldehyde diethyl acetal and ethyl malonyl chloride compounds, with the preferred process of the invention.

### DETAILED DESCRIPTION OF THE INVENTION

The process according to the invention for obtaining moguisteine essentially comprises three steps:
a) reacting guaiacol (2) with 2-Xmethyl-1,3-dioxolane (3), wherein X is a leaving group, to obtain 2-[(2-methoxyphenoxy)methyl]-1,3-dioxolane (4);
b) reacting the obtained 2-[(2-methoxyphenoxy)methyl]-1,3-dioxolane (4) with cysteamine (5) in the presence of an acid to obtain (R,S)-2-[(2-methoxyphenoxy)methyl]-1,3- thiazolidine (6); and
c) reacting (R,S)-2-[(2-methoxyphenoxy)methyl]-1,3-thiazolidine (6) with monoethylmalonic acid (7) or a salt thereof, in the presence of a condensing agent, to obtain moguisteine (1).

Step a) of the process of the invention preferably takes place in high boiling solvents. More preferably it takes place in a solvent selected from the group consisting of 1-methoxy-2-propanol, N-methylpyrrolidone, dimethylformamide, dimethylacetamide, diglyme (bis(2-methoxyethyl)ether), ethyl cellosolve, methyl cellosolve, ethylene glycol; still more preferably the reaction solvent is 1-methoxy-2-propanol.

In step a) 2-Xmethyl-1,3-dioxolane (3), wherein X is a leaving group, is used. Preferably the leaving group is selected from halogen, mesylate, tosylate, triflate, being more preferably a halogen selected from the group consisting of chlorine, bromine, and still more preferably bromine.

Advantageously, the use of a cyclic diacetal, 2-Xmethyl-1,3-dioxolane (3) (more stable and more difficult to hydrolyze than linear diacetals such as dimethyl and diethyl), minimizes the presence of condensation by-products due to free aldehyde formation.

According to the invention, in the reaction mixture an inorganic base in the form of a fine powder, preferably K₂CO₃ is advantageously present.

The mole ratio of guaiacol (2) to 2-Xmethyl-1,3-dioxolane (3) is preferably in the range from 1:1 to 1:1.5, more preferably about 1:1.18. The reaction of step a) which leads to formation of the intermediate (4) can take place at a temperature between 100° and 140°C. At the end of the reaction of step a) the intermediate (4) is obtained as a crystalline solid by crystallization.

In an embodiment of the invention, preferred both from the economic and technical viewpoint because of the excellent stability/crystallization qualities of the formed intermediate (4), step a) is carried out in 1-methoxy-2-propanol at a temperature from 120°C to 129°C in a mole ratio of guaiacol (2) to 2-Xmethyl-1,3-dioxolane (3) of about 1:1.18.

Step a) of the process according to the invention therefore allows a new intermediate of formula 2-[(2-methoxyphenoxy)methyl]-1,3-dioxolane (4) to be obtained, which presents excellent stability and crystalline properties. In this respect, the cyclic intermediate obtained appears, at the end of the reaction, as a nearly white solid with a purity greater than 99%, which can be used as such in the subsequent step b). Advantageously, in order to be suitable for use in the next step for obtaining thiazolidine, it does not require long and expensive purifications, such as distillations at high vacuum, which instead were necessary for the intermediate 2-(2-methoxyphenoxy)acetaldehyde diethyl acetal obtained in step 1) of the prior art process. Moreover, it is obtained:
- in yields greater than 80% compared with the theoretical and, in the preferred embodiment, greater than 85%; and
- at a purity greater than 99%.

According to the invention, step b) comprises the reaction between the intermediate 2-[(2-methoxyphenoxy)methyl]-1,3-dioxolane (4) of step a) and cysteamine (5) in the presence of an acid to obtain (R,S)-2-[(2-methoxyphenoxy)methyl]-1,3-thiazolidine (6).

Cysteamine (5) is preferably in the form of a salt, more preferably as a hydrochloride or sulphate, and even more preferably a hydrochloride.

The mole ratio of the intermediate (4) to cysteamine (5) is preferably in the range from 1:1 to 1:1.5, more preferably about 1:1.18.

The acid of the reaction mixture is preferably concentrated hydrochloric acid. The reaction of step b) in its preferred embodiment is carried out advantageously in the presence of isopropanol and deionized water, but other solvents can be used such as methanol and water or ethanol and water.

Once the reaction of step b) is ended, thiazolidine is obtained by crystallization. The crude (R,S)-2-[(2-methoxyphenoxy)methyl]-1,3- thiazolidine (6) of step b) is obtained in a yield greater than 80%, preferably greater than 90%, compared to the theoretical, and at a purity greater than 99%.

Step c) for obtaining moguisteine (1) comprises the reaction between (R,S)-2-[(2-methoxyphenoxy)methyl]-1,3- thiazolidine (6) and monoethylmalonic acid (7) or a salt thereof in the presence of a condensing agent.

The salt of monoethylmalonic acid is preferably a salt of an alkali metal, more preferably it is potassium monoethyl malonate.

According to the invention monoethylmalonic acid (7) can be added as such or conveniently released in situ from one of its salts.

Some salts of monoethylmalonic acid are commercially available at high purity. For example potassium monoethyl malonate is commercially available with a purity greater than 99% and with a low content of dipotassium malonate. The salts of monoethylmalonic acid can be advantageously obtained by hydrolysis with a suitable diethylmalonate base. For example, potassium monoethyl malonate can be advantageously obtained by simple hydrolysis of diethylmalonate by way of a KOH solution in ethanol.

The reaction between monoethylmalonic acid (7) and thiazolidine (6) takes place in the presence of a condensing agent. The condensing agent according to the invention can be N,N'-dicyclohexylcarbodiimide or N,N'-diisopropylcarbodiimide. Preferably it is N,N'-dicyclohexylcarbodiimide.

To speed up the reaction a catalyst can be advantageously added such as 1-hydroxybenzotriazole hydrate or N-hydroxy succinimide.

The mole ratio of thiazolidine (6) to monoethylmalonic acid is preferably in the range from 1:1 to 1:1.5, and more preferably about 1:1.10.

In a preferred embodiment of the invention, the reaction of step c) occurs between a salt of monoethylmalonic acid (7) and thiazolidine (6) in the presence of an acid such as concentrated hydrochloric acid and N,N'-dicyclohexylcarbodiimide used as a condensing agent.

Moguisteine (1) is obtained in yields greater than 90% compared to the theoretical, with a purity greater than 99%.

In the most preferred embodiment of the invention, the reaction of step c) takes place between thiazolidine (6) and potassium monoethyl malonate (7) in the presence of concentrated hydrochloric acid, N,N'-dicyclohexylcarbodiimide and ethyl acetate, and leads to the formation of moguisteine, potassium chloride and 1,3-dicyclohexylurea. After separation of the unwanted products by filtration, moguisteine is obtained in very high yields at a purity greater than 99.5%. Therefore, by means of the process of the invention, moguisteine is obtained in high yields, high purity and at low cost, avoiding the drawbacks of the prior art. In particular, referring to the annexed figure 1 that shows on the left the reagents used in the process of the prior art and on the right the reagents of the preferred process of the invention, the preparation of the new cyclic intermediate (4) and use of monoethylmalonic acid (7) or one of its salts, such as the potassium salt, in place of ethylmalonylchloride, have allowed a moguisteine product with purity greater than 99% to be obtained.

Furthermore the process of the invention is found to be more economical, on the one hand because of the lower raw material costs, and on the other hand because it does not require distillations under critical conditions to remove unwanted by-products.

The invention will now be described with reference to some embodiments given by way of non-limiting example.

### EXAMPLE 1

### Step a) Preparation of the intermediate (4) of formula 2-[(2-methoxyphenoxy)methyl]-1,3-dioxolane (4) in 1-methoxy-2-propanol

The raw materials were used in the amounts shown in the following Table 1.

**Table 1: Substances and amounts of Example 1**

| RAW MATERIALS | Density | g | ml | moles | mole ratio |
|---|---|---|---|---|---|
| Guaiacol | 1.129 | 297.6 | 263.6 | 2.40 | 1.00 |
| 1-methoxy-2-propanol | 0.920 | 607.2 | 660.0 | * | * |
| Potassium carbonate fine | | 497.6 | | 3.60 | 1.50 |
| powder | | | | | |
| 97% 2-bromomethyl-1,3-dioxolane | 1.628 | 475.2 | 291.9 | 2.76 | 1.15 |
| 50% KOH solution | 1.516 | 84.2 | 55.5 | 0.75 | 0.31 |
| Deionized water, 1 st portion | 1.00 | 660.0 | 660.0 | * | * |
| Deionized water, 2nd portion | 1.00 | 1320.0 | 1320.0 | * | * |
| Deionized water for washing | 1.00 | 2x500 | 2x500 | * | * |

Under nitrogen atmosphere, potassium carbonate fine powder and 1-methoxy-2-propanol were loaded into a perfectly dry 6 litre flask and the mass was placed under agitation. When the guaiacol had been loaded in a fine stream, the temperature rose spontaneously from 24°C to 42°C due to the exothermic salification and the mixture thickened considerably due to precipitation of the corresponding potassium salt. The mass was then heated to reflux (T = 121-123°C) and, having reached this temperature, 2-bromomethyl-1,3-dioxolane was added drop-wise over a period of 1 hour and 30 minutes, while maintaining always a good reflux. During the addition the presence of the reagent caused the boiling temperature of the mixture to rise to T = 127-129°C. The mixture was maintained at reflux for a total of about 24 hours from the start of bromodioxolane addition by dropping. As the reagent was consumed, it was noted that the boiling temperature tended to drop once more, returning to the initial value of T = 121-123°C.

The mixture was cooled to T = 20°±5°C and a sample was collected for monitoring the end of the reaction.

When the end of the reaction was confirmed, a 50% KOH solution was added to the mixture. As the temperature of the mixture rose spontaneously by about 10°C, it was cooled to T = 20°±25°C and the first portion of deionized water was added. Precipitation of the product was then awaited, possibly aided by seeding.

To complete the precipitation, the second portion of deionized water was then added. At the end of the addition, a dark green-grey fluid suspension was obtained which was maintained at T = 20°±5°C for at least 2 hours, then filtered and washed thoroughly with deionized water until the pH of the final wash liquid was 7-8.
481.0 g of a wet product of light hazel colour was obtained, equal to 425.0 g of dry product.
Theoretical yield = 504.54 g
Percentage yield relative to theoretical = 84.23%

The intermediate (4) obtained was then analysed and the following results were obtained:
HPLC purity = 99.81%
GC purity = 99.85%

The product was also subjected to the following analyses for its characterization.

The following results were obtained:
Melting point was 48°C.

| Elemental analysis: | | |
|---|---|---|
| Found | C = 62.83% | H = 6.76% |
| Theoretical | C = 62.85% | H = 6.71% |

| Mass analysis: | |
|---|---|
| CI+/MS | 211 [MH+], 210 [(MH+)-H]+ |

NMR Spectroscopy analysis:
¹³C NMR- APT
56.3 (OMe), 65.5 (CH2), 70.5 (CH2), 102.4 (C8-CH), 112.4 (CH), 121.1 (CH), 122.2 (CH), 148.6 (C), 150.1 (C)
¹H-NMR
3.84 (3H, s, OMe), 3.9-4.1 (4H, m, H-9/H-10), 4.07 (2H, d, J = 4 Hz, H-7), 5.33 (1H, t, J = 4 Hz, H-8), 6.7-7.0 (4H, m).

The analyses confirmed the structure of the intermediate (4)

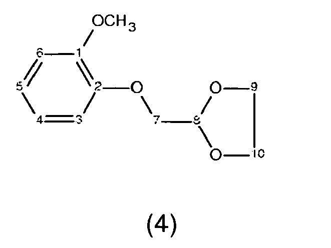

### EXAMPLE 2

### Step a) Preparation of the intermediate (4) of formula 2-[(2-methoxyphenoxy)methyl-1,3-dioxolane (4) in N-methylpyrrolidone

The following raw materials were used in the amounts shown in Table 2.

**Table 2: Substances and amounts used in Example 2**

| RAW MATERIALS | Density | g | ml | moles | mole ratio |
|---|---|---|---|---|---|
| Guaiacol | 1.129 | 99.2 | 87.9 | 0.80 | 1.00 |
| N-methylpyrrolidone | 1.028 | 514.0 | 500.0 | * | * |
| Potassium carbonate fine powder | | 221.14 | | 1.60 | 2.00 |
| 97% 2-bromomethyl-1,3-dioxolane | 1.628 | 158.4 | 97.3 | 0.92 | 1.15 |
| Deionized water for quenching | 1.00 | 1400.0 | 1400.0 | | |
| Toluene for extraction | 0.865 | 692.0 | 800.0 | | |
| Deionized water, 1 st wash | 1.00 | 800.0 | 800.0 | | |
| 30% NaOH solution | 1.335 | 50.0 | 37.5 | | |
| Deionized water, 2nd wash | 1.00 | 800.0 | 800.0 | | |
| 30% NaOH solution | 1.335 | 50.0 | 37.5 | | |
| Deionized water, 3rd wash | 1.00 | 800.0 | 800.0 | | |
| 30% NaOH solution | 1.335 | 50.0 | 37.5 | | |
| Carbon | | 5.0 | | | |
| Toluene for washing panel | 0.865 | 86.5 | 100.0 | | |

Under nitrogen atmosphere, potassium carbonate fine powder, guaiacol and N-methylpyrrolidone were loaded into a 3 litre flask at ambient temperature. The mixture was subjected to agitation and the temperature brought to 135°±5°C; having reached this temperature, 2-bromomethyl-1,3-dioxolane was added drop-wise over a period of at least 2 hours while keeping within the temperature range. At the end of the addition, the reaction was allowed to proceed at a temperature of 135°±5°C for a further 8 hours. (Total 2-bromomethyl-1,3-dioxolane addition time and reaction time was about 10 hours).

The mixture was then cooled to a temperature of about 25°±5°C and a sample was collected for monitoring the end of the reaction.

When the end of the reaction was confirmed, 1400 ml of quenching water and toluene for extraction were then added to the mixture, which was heated to a temperature of 45°±5°C. Agitation was stopped and the phases were allowed to separate for around 1 hour.

The lower aqueous phase, black in colour, was discarded and the organic phase was subjected to 3 washes with water/NaOH maintaining the temperature at 45°±5°C, and allowing it to separate for about 30 minutes. The organic extract, pale amber in colour, was cooled to T = 25°±5°C and treated with carbon. The extract was then filtered on a dicalite panel to obtain a perfectly clear yellow ochre filtrate; the panel was washed with toluene so as to combine the wash solution with the main filtrate.

The toluenic solution was transferred into a clean 2 litre flask, equipped for distillation. The distillate was concentrated under vacuum with a bath temperature of about 60°±5°C until a residue formed.

The obtained intermediate (4) appeared as a dense amber liquid which solidified on cooling.
252.8 g of a dark yellow concentrate were obtained which yielded 153 g of crystalline product at 98% purity.
Theoretical yield = 168.18 g.
Percentage yield relative to theoretical = 91.15%.

### EXAMPLES 3-31

### Step a) Preparation of the intermediate (4) of formula 2-[(2-methoxyphenoxy)methyl-1,3-dioxolane (4)

By following the same procedure as shown in example 1, but using the amounts, reaction solvent, times and temperatures indicated in Table 3, the intermediate (4) was obtained in the yields and purities indicated in Table 4 to follow.

**Table 3: Reaction conditions for examples 3-31**

| | Guaiacol g (mol) | K₂CO₃ g (mol) | 2-BrMe-1,3-dioxolane g (mol) | Solvent type (ml) | T_{reaction} (°C) | Time (hours) |
|---|---|---|---|---|---|---|
| Ex.3 | 49.6 (1) | 66.34(1.2) | 79.2 (1.18) | 1-methoxy-2-propanol (110) | 123/124 | 16 |
| Ex.4 | 49.6(1) | 82.92 (1.5) | 79.2 (1.18) | 1-methoxy-2-propanol (110) | 124/128 | 18 |
| Ex.5 | 49.6(1) | 110.56 (2) | 79.2 (1.18) | 1-methoxy-2-propanol (165) | 122/125 | 17 |
| Ex.6 | 148.8(1) | 331.7(2) | 236.5(1.18) | 1-methoxy-2-propanol (495) | 123/126 | 20 |
| Ex.7 | 148.8(1) | 199.02(1.2) | 236.5(1.18) | 1-methoxy-2-propanol (330) | 123/125 | 20 |
| Ex.8 | 148.8(1) | 199.02(1.2) | 236.5(1.18) | 1-methoxy-2-propanol (330) | 123/126 | 20 |
| Ex.9 | 148.8(1) | 199.02(1.2) | 236.5( 1.18) | 1-methoxy-2-propanol (330) | 123/126 | 20 |
| Ex.10 | 49.6(1) | 66.34(1.2) | 79.2 (1.18) | 1-methoxy-2-propanol (110) | 124 | 20 |
| Ex.11 | 49.6(1) | 66.34(1.2) | 79.2(1.18) | 1-methoxy-2-propanol (150) | 124 | 20 |
| Ex.12 | 49.6(1) | 66.34(1.2) | 79.2 (1.18) | 1-methoxy-2-propanol (200) | 124 | 20 |
| Ex.13 | 148.8(1) | 199.02(1.2) | 236.5(1.18) | 1-methoxy-2-propanol (330) | 125 | 20 |
| Ex.14 | 148.8(1) | 331.7(2) | 236.5(1.18) | 1-methoxy-2-propanol (495) | 129 | 20 |
| Ex.15 | 99.2(1) | 132.68(1.2) | 157.7(1.18) | 1-methoxy-2-propanol(400) | 126/127 | 20 |
| Ex.16 | 99.2(1) | 221.14(2) | 157.7(1.18) | 1-methoxy-2-propanol(400) | 126/127 | 20 |
| Ex.17 | 99.2(1) | 132.7(1.2) | 158 (1.18) | 1-methoxy-2-propanol(220) | 126 | 20 |
| Ex.18 | 99.2(1) | 132.7(1.2) | 158(1.18) | 1-methoxy-2-propanol(220) | 128 | 20 |
| Ex.19 | 49.6(1) | 66.34(1.2) | 79.2(1.18) | Dimethylformamide (110) | 127 | 10 |
| Ex.20 | 49.6 (1) | 66.34(1.2) | 79.2 (1.18) | Dimethylacetamide (110) | 128-132 | 10 |
| Ex.21 | 49.6(1) | 66.34(1.2) | 79.2 (1.18) | Diglyme(110) | 130-138 | 20 |
| Ex.22 | 49.6(1) | 66.34(1.2) | 79.2(1.18) | Ethylcellosolve (110) | 132 | 20 |
| Ex.23 | 49.6(1) | 66.34(1.2) | 79.2 (1.18) | Ethylene glycol (110) | 132 | 20 |
| Ex.24 | 49.6 (1) | 66.34(1.2) | 79.2 (1.18) | Methylcellosolve (110) | 125-126 | 17 |
| Ex.25 | 99.2(1) | 132.7(1.2) | 158.4(1.18) | 1-methoxy-2-propanol(220) | 128 | 20 |
| Ex.26 | 99.2(1) | 132.7(1.2) | 158.4(1.18) | 1-methoxy-2-propanol(220) | 131 | 20 |
| Ex.27 | 148.8(1) | 199.02(1.2) | 237.68(1.18) | 1-methoxy-2-propanol(330) | 128/131 | 24 |
| Ex.28 | 49.6(1) | 82.92 (1.5) | 79.2(1.18) | 1-methoxy-2-propanol(110) | 125/127 | 16 |
| Ex.29 | 49.6(1) | 82.92 (1.5) | 79.2(1.18) | 1-methoxy-2-propanol(110) | 123/126 | 20 |
| Ex.30 | 148.8(1) | 199.02 (1.2) | 237.6 (1.18) | 1-methoxy-2-propanol(330) | 124/129 | 24 |
| Ex.31 | 148.8(1) | 248.78(1.5) | 237.6 (1.18) | 1-methoxy-2-propanol(330) | 125/129 | 24 |

**Table 4: Yield and purity of the intermediate (4) obtained from examples 3-31**

| | Yield (g) | Theoretical yield (g) | % yield/theoretical yield | HPLC purity | GC purity |
|---|---|---|---|---|---|
| Ex.3 | 74.26 | 84.09 | 88.31 | 99.63 | 99.23 |
| Ex.4 | 72.62 | 84.09 | 86.35 | 99.61 | 99.56 |
| Ex.5 | 73.65 | 84.09 | 87.58 | 99.72 | 99.25 |
| Ex.6 | 223.0 | 251.78 | 88.56 | 99.70 | 99.46 |
| Ex.7 | 214.2 | 251.78 | 85.07 | 99.48 | 99.40 |
| Ex.8 | 207.4 | 251.78 | 82.37 | 99.65 | 99.28 |
| Ex.9 | 206.0 | 251.78 | 81.81 | 99.70 | 99.36 |
| Ex.10 | 70.34 | 84.09 | 83.65 | 99.89 | 99.02 |
| Ex.11 | 71.41 | 84.09 | 84.92 | 99.74 | 99.68 |
| Ex.12 | 67.77 | 84.09 | 80.59 | 99.83 | 99.50 |
| Ex.13 | 209.1 | 251.78 | 83.0 | 99.73 | 99.16 |
| Ex.14 | 206.0 | 251.78 | 81.82 | 99.68 | 99.14 |
| Ex.15 | 127.4 | 168.19 | 75.75 | 99.46 | - |
| Ex.16 | 138.8 | 168.19 | 82.52 | 99.71 | - |
| Ex.17 | 129.2 | 168.19 | 76.86 | 99.06 | 99.89 |
| Ex.18 | 127.45 | 168.19 | 75.78 | 99.12 | 99.55 |
| Ex.19 | 65.5 | 84.09 | 77.8 | 99.31 | 98.50 |
| Ex.20 | 67.9 | 84.09 | 80.75 | 95.94 | 99.34 |
| Ex.21 | 21.70 | 84.09 | 25.8 | 98.98 | 99.21 |
| Ex.22 | 66.6 | 84.09 | 79.2 | 98.60 | 99.38 |
| Ex.23 | 67.8 | 84.09 | 80.6 | 97.01 | 97.81 |
| Ex.24 | 74.50 | 84.09 | 88.59 | 99.48 | 99.82 |
| Ex.25 | 138.36 | 168.19 | 82.3 | 99.39 | 99.88 |
| Ex.26 | 136.75 | 168.19 | 81.3 | 98.76 | 99.88 |
| Ex.27 | 207 | 251.78 | 82.15 | 99.87 | 99.87 |
| Ex.28 | 72 | 84.09 | 85.71 | 99.95 | 99.78 |
| Ex.29 | 72.6 | 84.09 | 86.43 | 99.86 | 99.89 |
| Ex.30 | 212.3 | 251.78 | 84.32 | 99.76 | 99.78 |
| Ex.31 | 217.1 | 251.78 | 86.22 | 99.77 | 99.77 |

Therefore, in all the examples the intermediate (4) is obtained in high yields and at purities greater than 99%. According to the present invention, the intermediates of examples 1, 28, 29 and 31 had the best appearance and quality.

### EXAMPLE 32

### Step b) Preparation of (R,S)-2-[(2-methoxyphenoxy)methyl]-1,3-thiazolidine (6)

The raw materials were used in the amounts indicated in Table 5 below.

**Table 5: Substances and amounts of example 32**

| RAW MATERIALS | Density | g | ml | moles | mole ratio |
|---|---|---|---|---|---|
| 2-[(2-methoxyphenoxy)methyl]-1,3- | | 152.8 | | 0.7268 | 1.00 |
| dioxolane (4) | | | | | |
| Cysteamine hydrochloride | | 97.4 | | 0.8576 | 1.18 |
| Isopropanol | 0.785 | 359.5 | 458.0 | | |
| Deionized water | 1.00 | 153.0 | 153.0 | | |
| Concentrated hydrochloric acid | 1.20 | 12.0 | 10.0 | | |
| Deionized water for precipitation | 1.00 | 764.0 | 764.0 | | |
| 30% NaOH solution | 1.335 | 121.2 | 90.7 | 0.909 | 1.25 |
| Deionized water for washing crude product | 1.00 | 2x200 | 2x200 | | |

Under nitrogen atmosphere, the 2-[(2-methoxyphenoxy)methyl]-1,3-dioxolane as such obtained in example 2, cysteamine hydrochloride, isopropanol, deionized water and concentrated hydrochloric acid were loaded into a 1 litre flask. The mixture was then agitated and heated and the solution obtained (usually yellow) was brought to reflux (Ti = 79°/80°C). Having reached reflux, the solution was maintained under reflux for at least 8 hours. The mixture was then cooled to T = 25±5°C and sampling was carried out to establish that the reaction was complete. Simultaneously, deionized water for precipitation and a 30% NaOH solution were introduced into a 2 litre flask, and the solution was cooled to T = 0±5°C.

On confirmation that the reaction was complete, the hydrochloride solution was transferred over a period of 10 minutes into the precipitation flask. During the addition, the temperature rose spontaneously to 13/15°C and the mixture was again brought to T = 0±5°C.

Initially the product separated in glue-like form which then tended to grain very well. To promote crystallization of thiazolidine, a seed was used (thiazolidine is low melting like moguisteine).

When the product was well crystallized, the mixture was again brought to T = 0±5°C, maintaining the suspension under these conditions for at least 8 hours before filtering.

The crude product obtained was then filtered off and washed thoroughly with deionized water.
189.25 g of a white product with yellowish hues was obtained, which was oven dried under vacuum at T = 35°±5°C for about 24 hours.
Yield = 139.0 g of a dry product, barely yellow in colour
Theoretical yield = 163.75 g
Percentage yield on the theoretical = 84.88%
(R,S)-2-[(2-methoxyphenoxy)methyl]-1,3- thiazolidine (6) was then analysed to obtain the following results.
HPLC purity = 99.791%
HClO₄ titre = 99.34%

### EXAMPLES 33-50

### Step b) Preparation of (R,S)-2-[(2-methoxyphenoxy)methyl]-1,3-thiazolidine (6)

By following the same procedure and using the same reagents indicated in example 31, but using different amounts of the intermediate (4) obtained from some of examples 1,3-30, (R,S)-2-[(2-methoxyphenoxy)methyl]-1,3-thiazolidine (6) was obtained in the yields and purities as indicated in Table 6 below.

**Table 6: Amounts of the intermediate (4) and yields and purities of thiazolidine (6)**

| | Intermediate (4) (g) | Thiazolidine (6) yield (g) | Thiazolidine (6) theoretical yield (g) | % yield/ theoretical yield | HClO₄ titre | HPLC purity |
|---|---|---|---|---|---|---|
| Ex.33 | 200 of Ex.1 | 196.8 | 214.26 | 91.85 | 99.25 | 99.90 |
| Ex.34 | 70 of Ex.3 | 71.4 | 75 | 95.2 | 99.92 | 99.89 |
| Ex.35 | 70 of Ex.4 | 61.4 | 75 | 81.86 | 100.68 | 99.76 |
| Ex.36 | 70 of Ex.5 | 68.7 | 75 | 91.6 | 98.87 | 99.79 |
| Ex.37 | 210 of Ex.6 | 205.0 | 225.3 | 91.0 | 99.45 | 99.81 |
| Ex.38 | 200 of Ex.7 | 191.8 | 214.26 | 89.50 | 99.91 | - |
| Ex.39 | 200 of Ex.8 | 191.9 | 214.26 | 89.56 | 99.12 | 99.905 |
| Ex.40 | 200 of Ex.9 | 194.0 | 214.26 | 90.5 | 99.26 | 99.912 |
| Ex.41 | 65 of Ex.10 | 56.1 | 69.66 | 80.53 | 99.13 | 99.854 |
| Ex.42 | 65 of Ex.11 | 61.5 | 69.66 | 88.3 | 99.35 | 99.906 |
| Ex.43 | 200 of Ex.13 | 192.0 | 214.26 | 89.61 | 99.86 | 99.93 |
| Ex.44 | 200 of Ex.14 | 192.65 | 214.26 | 89.91 | 99.86 | 99.936 |
| Ex.45 | 100 of Ex.15 | 90.05 | 107.17 | 84.0 | 99.62 | 99.86 |
| Ex.46 | 62.5 of Ex.17 | 58.2 | 66.98 | 86.89 | 99.30 | 99.73 |
| Ex.47 | 100 of Ex.18 | 95.98 | 107.17 | 89.39 | 99.28 | 99.66 |
| Ex.48 | 100 of Ex.25 | 93.5 | 107.17 | 87.24 | 99.69 | 99.55 |
| Ex.49 | 100 of Ex.26 | 91.7 | 107.17 | 85.56 | 99.73 | 99.68 |
| Ex.50 | 70 of Ex.28 | 68.6 | 75.03 | 91.4 | 99.32 | 99.73 |

Step b) of the invention which uses as reagent the new cyclic intermediate (4) allows thiazolidine (6) to be obtained in yields higher than 80% and purities greater than 99%.

### EXAMPLE 51

### Step c) Preparation of moguisteine

The raw materials were used in the amounts indicated in Table 7 below.

**Table 7: Substances and amounts of Example 51**

| RAW MATERIALS | Density | grams | ml | moles | mole ratio |
|---|---|---|---|---|---|
| Dry crude thiazolidine of example 33 | | 50.0 | | 0.222 | 1.00 |
| Ethyl acetate | 0.902 | 225.5 | 250.0 | * | * |
| 95% monoethylmalonic acid | 1.119 | 33.93 | 30.3 | 0.244 | 1.10 |
| Ethyl acetate for dissolving DCC (N,N'-dicyclohexylcarbodiimide) | 0.902 | 225.5 | 250.0 | * | * |
| N,N'- dicyclohexylcarbodiimide | | 51.25 | | 0.244 | 1.10 |
| AcOEt for washing precipitated DCU (N,N'-dicyclohexylurea) | 0.902 | 90.2 | 100.0 | * | * |
| 50% acetic acid solution | | | 20.0 | | |
| 20% acetic acid solution | | | 100.0 | | |
| Carbon | | 2.5 | | | |
| ethyl acetate for washing panel | 0.902 | 45.1 | 50.0 | | |
| Acetone for dissolving the concentrate | 0.791 | 300.6 | 380.0 | | |
| Deionized water for precipitation | 1.00 | 460.0 | 460.0 | | |
| Deionized water for final wash | 1.00 | | 2x100.0 | * | * |

Under nitrogen atmosphere, thiazolidine of example 33, monoethylmalonic acid (colourless liquid) and the first portion of ethyl acetate were loaded into a one litre flask at ambient temperature. Agitation was applied and the mixture was cooled to Ti = 0°±5°C. Simultaneously, a solution of DCC (N,N'-dicyclohexylcarbodiimide) in ethyl acetate was prepared in a second container, protecting it from moisture. When T = 0°±5°C was attained, DCC (N,N'-dicyclohexylcarbodiimide) solution was added drop-wise over at least four hours. At the end of the addition the mass was left to react at Ti = 0°±5°C for about two hours. After checking the end of the reaction by means of thin layer chromatography, 50% acetic acid was added and the solution maintained under agitation overnight at Ti = 0°±5°C. The following morning, DCU (N,N'-dicyclohexylurea) was filtered off, washing thoroughly with ethyl acetate, the wash being added to the main filtrate.

The organic phase was treated under agitation with 20% acetic acid solution and warmed to 30-35°C to promote separation. On reaching this temperature agitation was stopped and the solution left to separate for about one hour. The acid aqueous phase (lower) was removed, the organic phase was decolorized with carbon and filtered over dicalite to obtain a perfectly clear solution, straw-yellow in colour.

The filtrate was concentrated under vacuum at bath T = 50°±5°C until a residue was obtained; the residue was dissolved in 380 ml of acetone at ambient temperature and transferred into a 1 litre flask.

The acetonic solution of moguisteine was cooled to T = 0°±5°C, and once this temperature was reached, 380 ml of water for precipitation were added over one hour; towards the end of the addition, the solution was seeded with moguisteine. After 15/30 minutes the product, that initially separated in oily glue-like form around the walls of the flask, crystallized and grained very well. Once the product had crystallized, the remaining 80 ml of the water were loaded.

The suspension was then maintained at Ti = 0±5°C for at least eight hours and left in the laboratory overnight under agitation.

The product was then filtered off and washed thoroughly with deionized water. 81.30 g of a nearly white wet product were obtained.

The product was oven dried at T = 35°±5°C until a KF value of <0.1% was attained.
68.65 g of a nearly white dry product were obtained.
Theoretical yield = 75.32 g
Percentage yield compared with theoretical = 91.14%
HPLC purity = 99.76%

### EXAMPLE 52

### Step c) Preparation of moguisteine

The raw materials were used in the amounts indicated in Table 8 below.

**Table 8: Substances and amounts of example 52**

| RAW MATERIALS | Density | g | ml | moles | mole ratio |
|---|---|---|---|---|---|
| Crude thiazolidine of example 33 | | 50.0 | | 0.222 | 1.00 |
| Acetone | 0.791 | 197.8 | 250.0 | * | * |
| 1-hydroxybenzoatetriazole hydrate | | 0.5 | | | |
| Potassium monoethyl malonate | | 41.5 | | 0.244 | 1.10 |
| Conc. hydrochloric acid (33.0%) | 1.20 | 24.5 | 20.4 | 0.222 | 1.00 |
| Acetone for dissolving DCC (N,N'-dicyclohexylcarbodiimide) | 0.791 | 197.8 | 250.0 | * | * |
| N,N'-dicyclohexylcarbodiimide | | 51.25 | | 0.244 | 1.10 |
| Acetone for washing precipitated DCU (N,N-dicyclohexylurea) | 0.791 | 110.7 | 140.0 | * | * |
| Carbon | | 2.5 | | | |
| Acetone for washing panel | 0.791 | 63.3 | 80.0 | | |
| Deionized water for precipitation | 1.00 | 456.0 | 456.0 | | |
| Deionized water for final wash | 1.00 | | 2x100.0 | * | * |

Under nitrogen atmosphere, (R,S)-2-[(2-methoxyphenoxy)methyl]-1,3-thiazolidine of example 33, monopotassium salt, 1-hydroxybenzoatetriazole hydrate (catalyst) and the first portion of acetone solvent were loaded into a 1 litre flask at ambient temperature. The suspension was cooled to T = 0°±5°C and concentrated hydrochloric acid was added drop-wise over about 1 hour (in this step exothermy was observed during the whole addition, the temperature rising spontaneously to 18°-20°C). Simultaneously, a solution of DCC (N,N'-dicyclohexylcarbodiimide) in acetone was prepared in a second container, protecting it from moisture. When the white suspension attained T = 0°±5°C, the DCC (N,N'-dicyclohexylcarbodiimide) solution was added drop-wise over at least 3 hours. At the end of the addition, the suspension was left at T = 0±5°C for about 2 hours. After checking the end of the reaction by means of thin layer chromatography (TLC), DCU (N,N'-dicyclohexylurea) was filtered off, washed thoroughly with acetone, the wash solution being added to the main filtrate.

The solution was concentrated under vacuum at bath T = 50°±5°C to a final volume of about 300 ml, and was left overnight at ambient temperature.

The dark yellow solution obtained was treated for 10 minutes with 5.0 g of carbon. The solution was then filtered on a dicalite panel to obtain a perfectly clear yellow solution which was transferred into a 1 litre flask. The filter was washed with 80 ml of acetone, and the solution after washing was combined with the main filtrate (300 ml + 80 ml = about 380 ml in total).

The acetonic solution of moguisteine was cooled to T = 0°±5°C and over 1 hour 380 ml of water for precipitation were added; towards the end of the addition, the solution was seeded with moguisteine. After 15-30 minutes the product, which initially separated in oily glue-like form around the walls of the flask, crystallized and grained very well. Once the product had crystallized, the remaining 76 ml of the water were introduced (380 ml + 76 ml = 456 ml; 380 ml acetone/ 456 ml of water acetone/ water ratio = 1.0:1.2)

The suspension was maintained at T = 0°±5°C for at least 8 hours and left overnight in the laboratory under agitation.

The product was then filtered off and washed thoroughly with deionized water. 94.4 g of a nearly white product were obtained.

The product obtained was oven dried at 35°±5°C.
69.5 g of a white dry powder with yellowish hues were obtained Theoretical yield = 75.32 g
Percentage yield compared with theoretical yield = 92.27%
HPLC purity attained was 99.81%

The test was repeated another four times, thus obtaining the following purity values respectively: 99.83%, 99.73%, 99.82%, 99.81 %.

The purity of moguisteine obtained from the process of the invention was so high as not to require a subsequent purification step.

### EXAMPLE 53

### Step c) Preparation of moguisteine

The raw materials in the amounts indicated in Table 9 below were used.

**Table 9: Substances and amounts of example 53**

| RAW MATERIALS | Density | g | ml | moles | mole ratio |
|---|---|---|---|---|---|
| Dry crude thiazolidine of example 33 | | 50.0 | | 0.222 | 1.00 |
| Ethyl acetate | 0.902 | 225.5 | 250.0 | * | * |
| Potassium monoethyl malonate | | 41.5 | | 0.244 | 1.10 |
| Conc. hydrochloric acid (33.0%) | 1.20 | 24.5 | 20.4 | 0.222 | 1.00 |
| Ethyl acetate for dissolving (N,N'-dicyclohexylcarbodiimide) | DCC 0.902 | 225.5 | 250.0 | * | * |
| N,N'- dicyclohexylcarbodiimide | | 51.25 | | 0.244 | 1.10 |
| AcOEt for washing precipitated DCU (N,N'-dicyclohexylurea) | 0.902 | 90.2 | 100.0 | * | * |
| 50% acetic acid solution | | | 20.0 | | |
| 20% acetic acid solution | | | 100.0 | | |
| Carbon | | 2.5 | | | |
| Ethyl acetate for washing panel | 0.902 | 45.1 | 50.0 | | |
| Acetone for dissolving the concentrate | 0.791 | 300.6 | 380.0 | | |
| Deionized water for precipitation | 1.00 | 460.0 | 460.0 | | |
| Deionized water for final wash | 1.00 | | 2x100.0 | * | * |

Under nitrogen atmosphere, thiazolidine of example 33, monopotassium salt and the first portion of the ethyl acetate were loaded into a one litre flask at ambient temperature. Agitation was applied and the suspension was cooled to T = 0°±5°C. When said temperature was attained, concentrated hydrochloric acid was added drop-wise over about one hour (in this step exothermy was observed during the whole of the addition, the temperature rising spontaneously to 18°-20°C). Simultaneously, a solution of DCC (N,N'-dicyclohexylcarbodiimide) in ethyl acetate was prepared in a second container, protecting it from moisture. When the white suspension attained T = 0°±5°C, the DCC (N,N'-dicyclohexylcarbodiimide) solution was added drop-wise over at least four hours. At the end of the addition the mass was left to react at Ti = 0°±5°C for about two hours. After checking the end of the reaction by means of thin layer chromatography, 50% acetic acid was added and the mass maintained under agitation overnight at T = 0°±5°C. The following morning, DCU (N,N'-dicyclohexylurea) was filtered off, washing thoroughly with ethyl acetate, the wash solution being combined with the main filtrate.

The organic phase was treated under agitation with 20% acetic acid solution and warmed to 30°-35°C to promote separation. On reaching this temperature, agitation was stopped and the solution left to separate for about one hour. The acid aqueous phase (lower) was removed, the organic phase was decolorized with carbon and filtered over dicalite to obtain a perfectly clear solution, straw-yellow in colour.

The filtrate was concentrated under vacuum at bath T = 50°±5°C until a residue was obtained; the residue was dissolved in 380 ml of acetone at ambient temperature and transferred into a 1 litre flask.

The acetonic solution of moguisteine was cooled to T = 0°±5°C, and once this temperature was reached 380 ml of water for precipitation was added over one hour; towards the end of the addition, the solution was seeded with moguisteine. After 15/30 minutes the product, that initially separated in oily glue-like form around the walls of the flask, crystallized and grained very well. Once the product had crystallized, the remaining 80 ml of the water were added.

The suspension was then maintained at T = 0°±5°C for at least eight hours and left overnight in the laboratory under agitation.

The product was then filtered off and washed thoroughly with deionized water. 79.49 g of a nearly white wet product were obtained.

The product was oven dried at T = 35°±5°C until a KF value of <0.1% was attained.
67.83 g of a nearly white dry product were obtained.
Theoretical yield = 75.32 g
Percentage yield compared with theoretical = 90.05%
HPLC purity = 99.88%

## Claims

1. Process for the synthesis of moguisteine that is ethyl ester of (R,S)-3-[2-[(2-methoxyphenoxy)methyl]-1,3-thiazolidin-3-yl]-3-oxypropanoic acid, comprising the following steps:
a) reacting guaiacol (2) with 2-Xmethyl-1,3-dioxolane (3) wherein X is a leaving group, to obtain 2-[(2-methoxyphenoxy)methyl]-1,3-dioxolane (4): 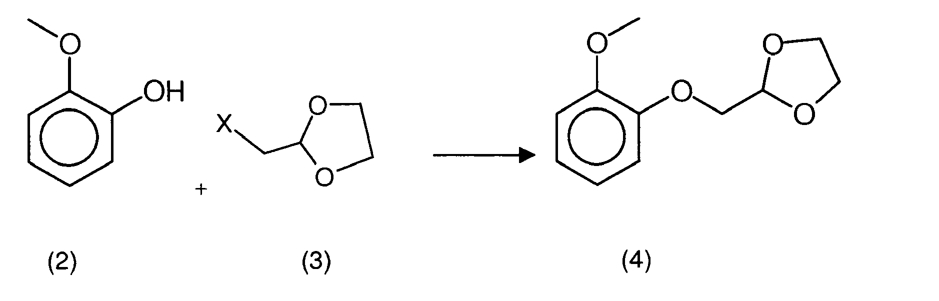
b) reacting 2-[(2-methoxyphenoxy)methyl]-1,3-dioxolane (4) obtained in step a) with cysteamine (5) in the presence of an acid to obtain (R,S)-2-[(2-methoxyphenoxy)methyl]-1,3-thiazolidine (6): 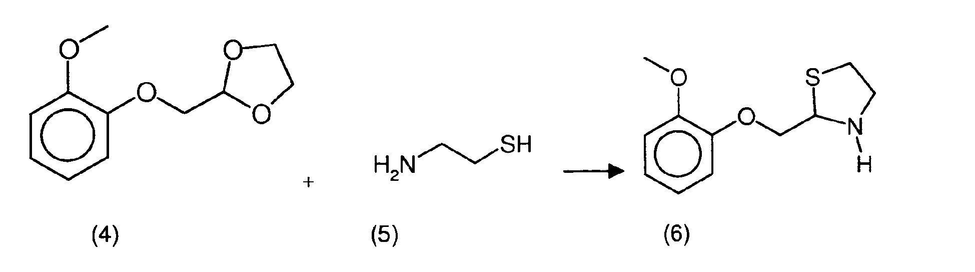
c) reacting (R,S)-2-[(2-methoxyphenoxy)methyl]-1,3-thiazolidine (6) with monoethylmalonic acid (7) or a salt thereof, in the presence of a condensing agent, to obtain moguisteine (1):

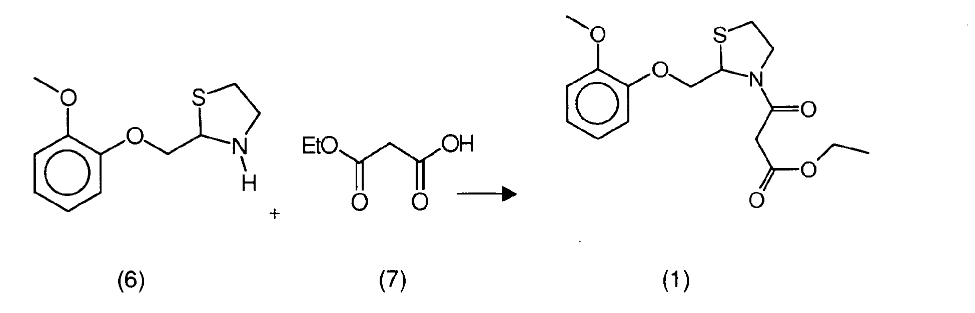

2. Process according to claim 1, wherein the leaving group X of 2-Xmethyl-1,3-dioxolane (3) of step a) is selected from the group consisting of halogen, mesylate, tosylate and triflate.

3. Process according to claim 2, wherein the halogen is selected from the group consisting of bromine and chlorine, being preferably bromine.

4. Process according to any one of claims from 1 to 3, wherein step a) takes place in a solvent selected from the group consisting of 1-methoxy-2-propanol, N-methylpyrrolidone, dimethylformamide, dimethylacetamide, diglyme (bis(2-methoxyethyl)ether), ethyl cellosolve, methyl cellosolve, ethylene glycol.

5. Process according to claim 4, wherein the solvent is 1-methoxy-2-propanol.

6. Process according to any one of claims from 1 to 5, wherein the mole ratio of guaiacol (2) to 2-Xmethyl-1,3 dioxolane (3) in step a) is from 1:1 to 1:1.5, preferably 1:1.18.

7. Process according to any one of claims from 1 to 6, wherein the reaction of step a) takes place at temperatures in the range from 100° to 140°C.

8. Process according to any one of claims from 1 to 7, wherein in step a) K₂CO₃ in fine powder form is also added to the reaction.

9. Process according to any one of claims from 1 to 8, wherein step a) takes place in 1-methoxy-2-propanol, at a temperature from 120°C to 129°C, in a mole ratio of guaiacol (2) to 2-Xmethyl-1,3 dioxolane (3) of about 1:1.18.

10. Process according to any one of claims from 1 to 9, wherein in step b) the mole ratio of the intermediate (4) to cysteamine (5) is in the range from 1:1 to 1:1.5, being preferably about 1:1.18.

11. Process according to any one of claims from 1 to 10, wherein cysteamine (5) is in salt form.

12. Process according to claim 11, wherein cysteamine is in the form of a hydrochloride salt.

13. Process according to any one of claims from 1 to 12, wherein the acid of the reaction mixture of step b) is concentrated hydrochloric acid.

14. Process according to any one of claims from 1 to 13, wherein the reaction of step b) takes place in the presence of isopropanol and deionized water.

15. Process according to any one of claims from 1 to 14, wherein the condensing agent in step c) is N,N'-dicyclohexylcarbodiimide.

16. Process according to any one of claims from 1 to 15, wherein the mole ratio of thiazolidine (6) to monoethylmalonic acid (7) or a salt thereof in step c) is in the range from 1:1 to 1:1.5, being preferably about 1:1.10.

17. Process according to any one of claims from 1 to 16, wherein monoethylmalonic acid (7) salt is potassium monoethyl malonate.

18. Process according to any one of claims from 1 to 17, wherein the reaction of step c) takes place between potassium monoethyl malonate (7) and thiazolidine (6) in the presence of concentrated hydrochloric acid and N,N'-dicyclohexylcarbodiimide in ethyl acetate.

19. An intermediate of formula 2-[(2-methoxyphenoxy)methyl]-1,3-dioxolane (4):

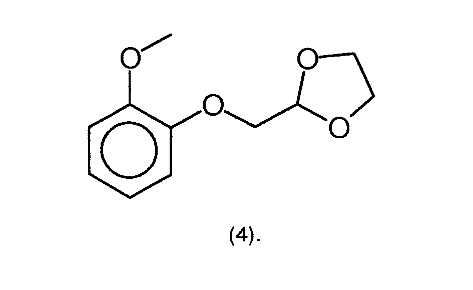

## Patentansprüche

1. Verfahren zur Synthese von Moguistein, welches Ethylester von (R,S)-3-[2-[(2-Methoxyphenoxy)methyl]-1,3-thiazolidin-3-yl]-3-oxypropansäure ist, welches die nachfolgenden Schritte umfasst:
a) Reagieren von Guajakol (2) mit 2-X-Methyl-1,3-dioxolan (3), worin X eine Abgangsgruppe ist, um 2-[(2-Methoxyphenoxy)methyl]-1,3-dioxolan (4) zu erhalten: 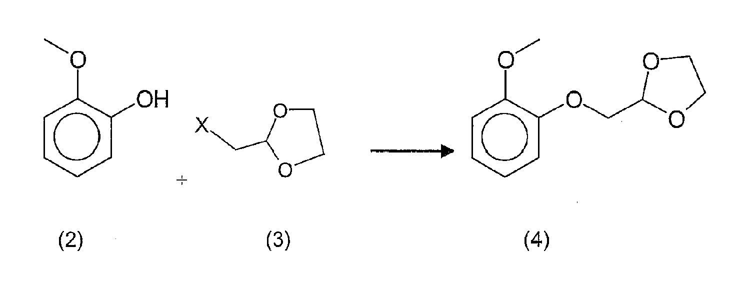
b) Regieren von in dem Schritt a) erhaltenem 2-[(2-Methoxyphenoxy)-methyl]-1,3-dioxolan (4) mit Cysteamin (5) in der Gegenwart von einer Säure, um (R,S)-2-[(2-Methoxyphenoxy)methyl]-1,3-thiazolidin (6) zu erhalten: 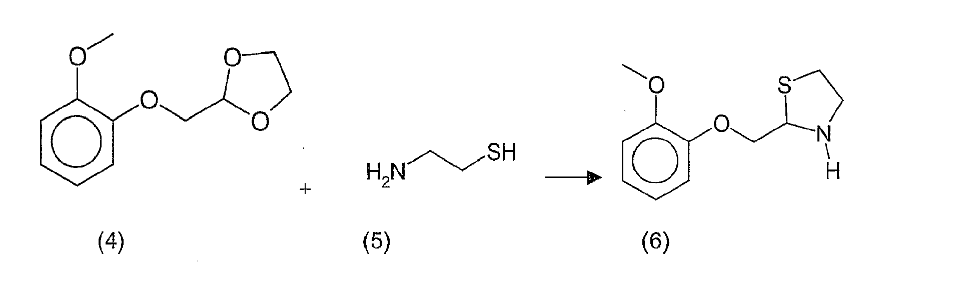
c) Reagieren von (R,S)-2-[(2-Methoxyphenoxy)methyl]-1,3-thiazolidin (6) mit Monoethylmalonsäure (7) oder einem Salz hiervon in der Gegenwart eines Kondensationsmittels, um Moguistein (1) zu erhalten:

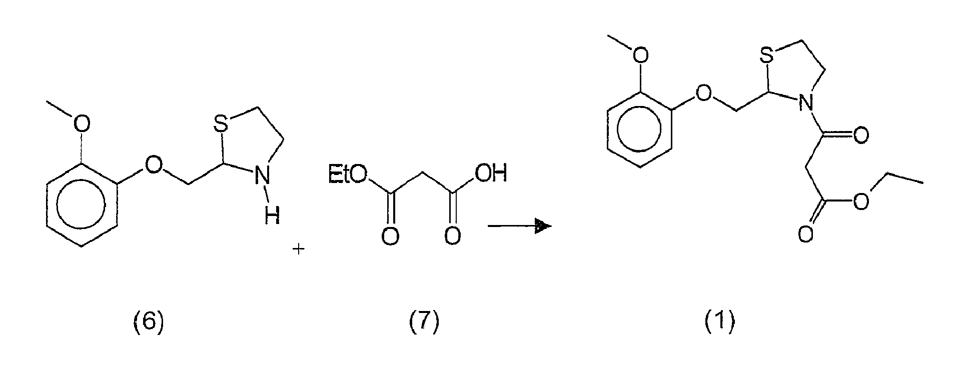

2. Verfahren nach Anspruch 1, wobei die Abgangsgruppe X von 2-XMethyl-1,3-dioxolan (3) von dem Schritt a) aus der Gruppe ausgewählt wird, welche aus Halogen, Mesylat, Tosylat und Triflat besteht.

3. Verfahren nach Anspruch 2, wobei das Halogen aus der Gruppe ausgewählt wird, welche aus Brom und Chlor besteht, wobei Brom bevorzugt ist.

4. Verfahren nach einem der Ansprüche 1 bis 3, wobei der Schritt a) in einem Lösungsmittel stattfindet, welches aus der Gruppe ausgewählt wird, welche aus 1-Methoxy-2-propanol, N-Methylpyrrolidon, Dimethylformamid, Dimethylacetamid, Diglyme-(bis(2-methoxyethyl)ether), Ethylcellosolve, Methlylcellosolve und Ethylenglykol besteht.

5. Verfahren nach Anspruch 4, wobei das Lösungsmittel 1-Methoxy-2-propanol ist.

6. Verfahren nach einem der Ansprüche 1 bis 5, wobei das Molverhältnis von Guajakol (2) zu 2-X-Methyl-1,3-dioxolan (3) in dem Schritt a) zwischen 1:1 1 und 1:1, 5, vorzugsweise 1:1,18, beträgt.

7. Verfahren nach einem der Ansprüche 1 bis 6, wobei die Reaktion des Schritts a) bei Temperaturen in einem Bereich zwischen 100° und 140°C stattfindet.

8. Verfahren nach einem der Ansprüche 1 bis 7, wobei in dem Schritt a) K₂CO₃ in feiner Pulverform ebenfalls zu der Reaktion zugegeben wird.

9. Verfahren nach einem der Ansprüche 1 bis 8, wobei der Schritt a) in 1-Methoxy-2-propanol bei einer Temperatur zwischen 120°C und 129°C in einem Molverhältnis von Guajakol (2) zu 2-X-Methyl-1,3-dioxolan (3) von ungefähr 1:1,18 stattfindet.

10. Verfahren nach einem der Ansprüche 1 bis 9, wobei in dem Schritt b) das Molverhältnis des Zwischenprodukts (4) zu Cysteamin (5) in einem Bereich zwischen 1:1 1 und 1:1, 5 liegt und vorzugsweise ungefähr 1:1,18 beträgt.

11. Verfahren nach einem der Ansprüche 1 bis 10, wobei das Cysteamin (5) in der Salzform vorliegt.

12. Verfahren nach Anspruch 11, wobei das Cysteamin in der Form eines Hydrochloridsalzes vorliegt.

13. Verfahren nach einem der Ansprüche 1 bis 12, wobei die Säure der Reaktionsmischung des Schritts b) konzentrierte Salzsäure ist.

14. Verfahren nach einem der Ansprüche 1 bis 13, wobei die Reaktion des Schritts b) in der Gegenwart von Isopropanol und von deionisiertem Wasser stattfindet.

15. Verfahren nach einem der Ansprüche 1 bis 14, wobei das Kondensationsmittel in dem Schritt c) N,N'-Dicyclohexylcarbodiimid ist.

16. Verfahren nach einem der Ansprüche 1 bis 15, wobei das Molverhältnis von Thiazolidin (6) zu Monoethylmalonsäure (7) oder einem Salz hiervon in dem Schritt c) in einem Bereich zwischen 1:1 1 und 1:1, 5 liegt und bevorzugt ungefähr 1:1,10 beträgt.

17. Verfahren nach einem der Ansprüche 1 bis 16, wobei das Monoethylmalonsäure(7)-Salz Kaliummonoethylmalonat ist.

18. Verfahren nach einem der Ansprüche 1 bis 17, wobei die Reaktion des Schritts c) zwischen Kaliummonoethylmalonat (7) und Thiazolidin (6) in der Gegenwart von konzentrierter Salzsäure und von N,N'-Dicyclohexylcarbodiimid in Ethylacetat stattfindet.

19. Zwischenprodukt gemäß der Formel 2-[(2-Methoxyphenoxy)methyl]-1,3-dioxolan (4):

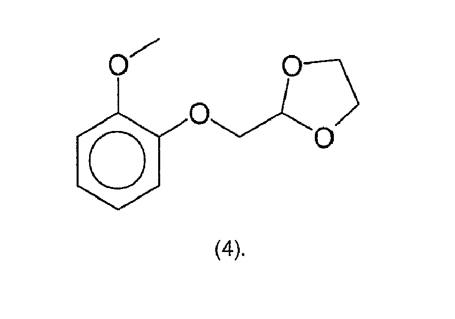

## Revendications

1. Procédé de synthèse de moguistéine qui est l'ester d'éthyle de l'acide (R,S)-3-[2-[(2-méthoxyphénoxy)méthyl]-1,3-thiazolidin-3-yl]-3-oxypropanoïque, comprenant les étapes suivantes consistant à :
a) faire réagir du guaïacol (2) avec du 2-Xméthyl-1,3-dioxolane (3) où X est un groupe partant, pour obtenir du 2-[(2-méthoxyphénoxy)méthyl]-1,3-dioxolane (4) : 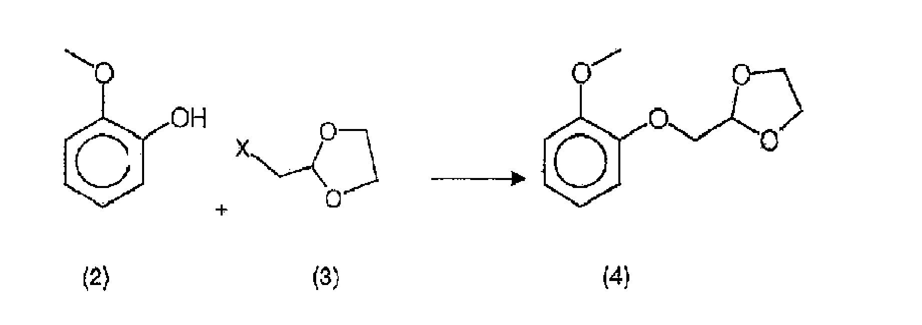
b) faire réagir du 2-[(2-méthoxyphénoy)méthyl]-1,3-dioxolane (4) obtenu à l'étape a) avec de la cystéamine (5) en présence d'un acide pour obtenir de la (R,S)-2-[(2-méthoxyphénoxy)méthyl]-1,3-thiazolidine (6) : 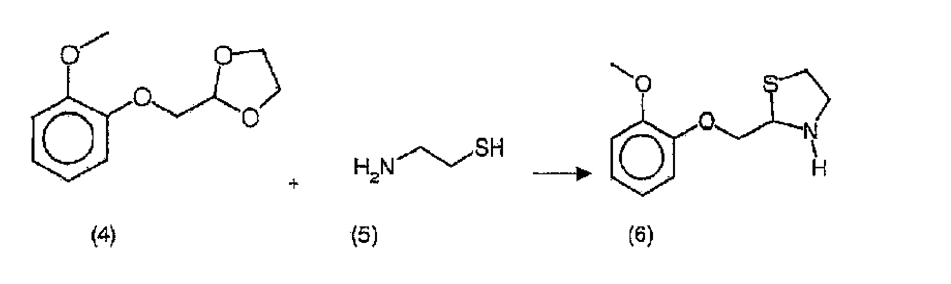
c) faire réagir de la (R,S)-2-[(2-méthoxyphénoxy)méthyl]-1,3-thiazolidine (6) avec de l'acide monoéthylmalonique (7) ou l'un de ses sels, en présence d'un agent de condensation, pour obtenir de la moguistéine (1) :

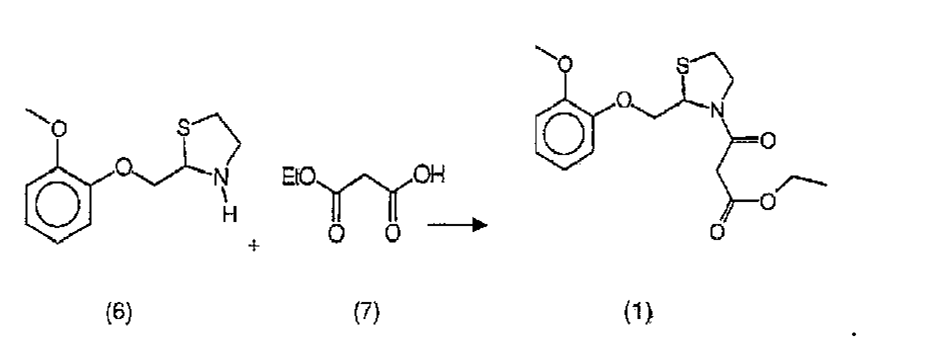

2. Procédé selon la revendication 1, dans lequel le groupe partant X de 2-X-méthyl-1,3-dioxolane (3) de l'étape a) est choisi dans le groupe constitué par un atome d'halogène, un groupe mésylate, tosylate et triflate.

3. Procédé selon la revendication 2, dans lequel l'atome d'halogène est choisi dans le groupe constitué par le brome et le chlore, le brome étant préféré.

4. Procédé selon l'une quelconque des revendications 1 à 3, dans lequel l'étape a) a lieu dans un solvant choisi dans le groupe constitué par le 1-méthoxy-2-propanol, la N-méthylpyrrilidone, le diméthylformamide, le diméthylacétamide, la diglyme, le bis(2-méthoxyéthyl)éther, l'éthyl cellosolve, le méthyl cellosolve et l'éthylène glycol.

5. Procédé selon la revendication 4, dans lequel le solvant est le 1-méthoxy-2-propanol.

6. Procédé selon l'une quelconque des revendications 1 à 5, dans lequel le rapport en mole entre guaïacol (2) et 2-Xméthyl-1,3-dioxolane (3) dans l'étape a) est de 1 : 1 à 1 : 1,5, de préférence de 1 : 1,18.

7. Procédé selon l'une quelconque des revendications 1 à 6, dans lequel la réaction de l'étape a) a lieu à des températures dans la plage de 100°C à 140°C.

8. Procédé selon l'une quelconque des revendications 1 à 7, dans lequel dans l'étape a), du K₂CO₃ sous forme de poudre fine est également ajouté à la réaction.

9. Procédé selon l'une quelconque des revendications 1 à 8, dans lequel l'étape a) a lieu dans du 1-méthoxy-2-propanol, à une température de 120°C à 129°C, dans un rapport en mole entre guaïacol (2) et 2-Xméthyl-1,3-dioxolane (3) d'environ 1 : 1,18.

10. Procédé selon l'une quelconque des revendications 1 à 9, dans lequel dans l'étape b), le rapport en mole entre l'intermédiaire (4) et la cystéamine (5) est dans la plage de 1 : 1 à 1 : 1,5, de préférence d'environ 1 : 1,18.

11. Procédé selon l'une quelconque des revendications 1 à 10, dans lequel la cystéamine (5) est sous forme de sel.

12. Procédé selon la revendication 11, dans lequel la cystéamine est sous forme d'un sel de chlorhydrate.

13. Procédé selon l'une quelconque des revendications 1 à 12, dans lequel l'acide du mélange de réaction de l'étape b) est de l'acide chlorhydrique concentré.

14. Procédé selon l'une quelconque des revendications 1 à 13, dans lequel la réaction de l'étape b) a lieu en présence d'isopropanol et d'eau désionisée.

15. Procédé selon l'une quelconque des revendications 1 à 14, dans lequel l'agent de condensation dans l'étape c) est le N,N'-dicyclohexylcarbodiimide.

16. Procédé selon l'une quelconque des revendications 1 à 15, dans lequel le rapport en mole entre thiazolidine (6) et acide monoéthylmalonique (7) ou un sel de celui-ci dans l'étape c) est dans la plage de 1 : 1 à 1 : 1,5, de préférence d'environ 1 : 1,10.

17. Procédé selon l'une quelconque des revendications 1 à 16, dans lequel le sel de l'acide monoéthylmalonique (7) est le malonate de monoéthyle potassium.

18. Procédé selon l'une quelconque des revendications 1 à 17, dans lequel la réaction de l'étape c) a lieu entre du malonate de monoéthyle potassium (7) et de la thiazolidine (6) en présence d'acide chlorhydrique concentré et de N,N'-dicyclohexylcarbodiimide dans de l'acétate d'éthyle.

19. Intermédiaire de formule 2-[(2-méthoxyphénoxy)méthyl]-1,3-dioxolane (4) :
